# EUROPÄISCHE PATENTSCHRIFT

(11) **EP 0 444 118 B1**
(45) Veröffentlichungstag und Bekanntmachung des Hinweises auf die Patenterteilung: **29.06.1994**
(21) Anmeldenummer: 90900134.9
(22) Anmeldetag: 17.11.1989
(51) Int. Cl.: G01N 33/573, G01N 33/574, C07K 15/28, C12P 21/08, A61K 39/395

(54) **PYRUVATKINASE-ISOENZYME TYP M2 (TUMOR M2-PK) - SPEZIFISCHE ANTIKÖRPER/VERFAHREN ZU DEREN HERSTELLUNG UND DEREN VERWENDUNG**
PYRUVATE-KINASE-ISOENZYME-TYPE-M2 (TUMOUR M2-PK) SPECIFIC ANTIBODIES, PROCESS FOR PRODUCING THEM AND USE
ANTICORPS SPECIFIQUES A l'ISOENZYME DE LA PYRUVATE-KINASE DU TYPE M2 (M2-PK TUMORALE), PROCEDE DE FABRICATION ET APPLICATION

(30) Priorität: 17.11.1988 DE 3838900
(43) Veröffentlichungstag der Anmeldung: 04.09.1991
(73) Patentinhaber: SCHEBO TECH MEDIZINISCH-BIOLOGISCHE FORSCHUNGSGESELLSCHAFT M.B.H, D-35435 Wettenberg (DE)
(72) Erfinder: EIGENBRODT, Erich, D-6307 Linden (DE); REINACHER, Manfred, D-6300 Giessen (DE); SCHEEFERS-BORCHEL, Ursula, D-6301 Wettenberg 2 (DE); SCHEEFERS, Hans, D-6301 Wettenberg 2 (DE)
(74) Vertreter: Weickmann, Heinrich, Dipl.-Ing.
(86) Internationale Anmeldenummer: EP8901384
(87) Internationale Veröffentlichungsnummer: WO9005917

(56) Entgegenhaltungen:
- WO-A-86/00992
- WO-A-87/03094
- Biomed. Biochem. Acta, vol. 46, March 1987; M. HEINRICHS et al., pp. 223-228;
- European Journal of Cell Biology, vol. 47, 1988; H.E. VAN ERP et al., pp. 388-394;
- Virchows Arch (Cell Pathol), vol. 37, 1981; M. REINACHER et al., pp. 79-88;
- Chemical Abstracts, vol. 109, no. 25, 19 December 1988 (Columbus, Ohio, US); OUDE WEERNINK et al., p. 619, abstract no. 228157m;
- Biomed. Biochem. Acta, vol. 42 (1983), 11/12; E. EIGENBROT et al., pp. 278-282;

## Beschreibung

Die Erfindung betrifft ein Verfahren zur selektiven Bestimmung des menschlichen Pyruvatkinase(ATP: pyruvate 2-O- phosphotransferase, EC 2.7.1.40) -Isoenzym Typ M2 (Tumor-M2-PK) im Plasma, Blut oder Gewebe mit hochspezifischen Antikörpern, die hierfür benötigten Tumor-M2-PK-spezifisch bindefähigen und zwischen anderen Pyruvatkinase-Isoenzymen (M1,L und R-Typ) diskriminierenden Antikörper, sowie ihre Verwendung und ein sie enthaltendes Reagenz.

### Einleitung:

Krebserkrankungen gehören heute neben den Herz-Kreislauferkrankungen zu den häufigsten Todesursachen in den Ländern mit einer guten medizinischen Versorgung; hier ist etwa ein Viertel aller Todesfälle auf das Auftreten maligner Tumoren zurückzuführen. F.W. MacKay Cancer Mortality in the United States 1950-1977. National Cancer Department Monograph Bd. 59 U.S. Government Printing Office 1982; R.W. Miller und F.W. McKay, J. Am. Med. Ass. 251, 1567 (1984).
Es ist zu erwarten, daß dieser Prozentsatz bei allgemein zunehmender Lebenserwartung noch ansteigen wird.
Die Behandlung von Krebserkrankungen ist vielschichtig, da es "den Krebs" nicht gibt, sondern über 100 verschiedene Typen bekannt sind. Wenngleich in den vergangenen Jahren wesentliche Erkenntnisse über die Krebs-Entstehung gewonnen werden konnten, so ist die Pathogenese maligner Tumoren immer noch in vielen Bereichen völlig ungeklärt. Es kann jedoch davon ausgegangen werden, daß Krebs zum einen durch Veränderungen von Genen hervorgerufen wird, die das Wachstum und die Zelldifferenzierung steuern (somatische Mutationstheorie) D. Harden: Progress in Cancer Research and Therapy Bd.III S.87. Raven Press, New York 1977 oder auf einer Fehlregulation der Genexpression (Mißdifferenzierungstheorie) A. Upton : Cancer, Prinziples and Practice of Oncology S. 33. J.B. Lippincott Company, Philadelphia Toronto 1982 beruht.

Weitgehend gesichert ist auch die Annahme, daß Krebs in begrenztem Maße durch Aktivierung viraler DNA oder RNA, die in das Genom der Wirtszelle als Oncogen eingebaut sind, verursacht wird. J.M. Bishop, Anual. Rev. Biochem. 52, 301 (1983); R.A. Weinberg Science 230, 770 (1985).

Ein direkter Zusammenhang, zwischen dem Einfluß natürlicher und künstlicher, chemischer oder physikalischer Noxen und der Entstehung von Krebs konnten in einigen Fällen zweifelsfrei nachgewiesen werden.( J.C. Acros, M.F. Argus und G.Wolf (Hrsg.): Chemical Induction of Cancer Bd. 1 Academic Press, New York 1968).

Zur Erkennung (Aufdeckung) eines malignen Geschehens im Organismus, oder zur Unterscheidung von "normalen" und transformierten Zellen ist es erforderlich biochemische Unterschiede auf molekularer Basis zwischen entarteten und normalen Zellen aufzufinden; hierbei kann die Differenzierung entweder in der Oberflächenstruktur (Unger H. Eibl und G.A. Nagel (Hrsg.): Die Zellmembran als Angriffspunkt der Tumortherapie Zuckschwerdt Verlag 1987) oder im Metabolismus der Zellen liegen (O. Bodansky Biochemistry of Human Cancer Academic Press, New York 1975). Es wurden einige biochemische Unterschiede zwischen "normalen" und malignen Zellen gefunden. Eine Ausnutzung dieser Unterschiede für eine generelle, selektive Tumortherapie ist bisher nicht gelungen, da sie nur in einigen Tumorarten gefunden weden und ihre Quantität nicht ausreichend ist.

Es ist jedoch bemerkenswert, daß einer der am weitesten verbreiteten Stoffwechselunterschiede zwischen normalen und malignen Zellen eine gesteigerte aerobe Glykolyserate bei malignen Zellen ist. Bereits 1930 hatte Warburg (O. Warburg: The Metabolism of Tumours Constable, London 1930)in in-vitro- Versuchen zeigen können, daß bei der Inkubation von Normalgewebe in einer Pufferlösung mit Glukose in Gegenwart von Sauerstoff keine oder fast keine Milchsäure gebildet wird, während unter gleichen Bedingungen im Tumorgewebe erhebliche Mengen Laktat entstehen.
Schon vorher hatten Cori und Cori beschrieben, daß die Applikation von Glukose an Mäusen mit einem Mamma-Carcinom zu vermehrter Milchsäure-bildung führt.( C.F. Cori und G.T. Cori, J. Biol. Chem. 64, 11 (1925); 65, 397 (1925).

Die erhöhte aerobe Glykolyse wurde bisher bei fast allen malignen Zellen tierischer und menschlicher Herkunft nachgewiesen.

### Stand der Wissenschaft und Technik

In der klinischen Chemie können Tumore durch den Nachweis von Enzymen (saure Phosphatase, gamma GT.. oder von Substanzen wie alpha-Fetoprotein, tissue polypeptid antigen, sialic acid und karzinoembryonales Antigen nachgewiesen werden. Herschmann, H.R Trends Biochem. Sci. 5, 82-84 (1980); Stugeon, C.M. Trends Biochem. Sci. 4, 121-123.(1979); Schuster, J., Livingston, A., Banjo, C., Silver, H.K.B., Freedman, S.O. and Gold, P. (1975); Silver, H.K.B., Gold, P., Feder, S. and Schuster, J. (1973); Björklund, B. Tumor Diagnostik 1, 9-20 (1979); Björklund B., Wiklund, B., Lüning, B., Anderson, K., Kallin, E. and Björklund V. Tumor Diagnostik 2, 78-84 (1980); Lüthgens, M. und Schlegel, G. Tumor Diagnostik 2, 63-77 (1980); Silver, K.B.H., Karim, K.A., Archibald, E.L. and Salinas, F.S. Cancer Res. 39, 5036-5042 (1979).Der Nachweis eines Isoenzyms der sauren Phosphatase im Blut deutet mit großer Sicherheit auf das Vorhandensein von Prostatatumoren und deren Knochenmetastasen hin. Die Spezifität dieser Aussage konnte durch die Einführung eines ELISA-Tests noch erhöht werden. Herschmann, H.R. Trends Biochem. Sci. 5, 82-84 (1980). Die gamma-Glutamyltransferase (gamma-GT)-Aktivität, die in präneoplastischen Leberfoci von Ratte und Maus erhöht ist, zeigt keine Korrellation mit dem Malignitätsgrad der Tumoren. Boelsterli, U. Trends Pharmacol. Sci. 1, 47-49 (1979). Das karzinoembryonale Antigen (CEA) findet sich bei einer Reihe von Tumoren erhöht, aber auch bei nicht-kanzeromatösen Erkrankungen. Das "tissue polypeptide antigen" (TPA) kann hauptsächlich als Hinweis für Mammatumore Verwendung finden. Björklund, B. Tumor Diagnostik 1, 9-20 (1979). Auch das alpha- Fetoprotein hat im wesentlichen bei der Diagnose und der Verlaufskontrolle von Leber- und Germinalzelltumoren Anwendung gefunden. Abelev, G.K. Cancer Res. 28, 1344-1350 (1968).; Abelev, G.K. Adv. Cancer Res. 14, 295-358; Sell, S., Becker, F. F., Leffert, H.L. and Watabe Cancer Res. 36, 4239-4249 (1976).

Alle diese Tests besitzen somit eine relativ hohe Gewebespezifität und können daher nicht generell zur Tumordiagnostik verwandt werden. Sie werden dementsprechend auch eher zur Therapiekontrolle bekannter Tumoren eingesetzt als zur Tumorsuche und -diagnostik. Koch, O.M. und Uhlenbruck, G. Med. Diagnostik 33, 29-38 (1983).

Im Gegensatz hierzu zeigen Untersuchungen, daß die Tumor-M2-PK in allen Tumoren, mit Ausnahme von solchen Tumoren, die von Pyruvatkinase Typ L enthaltenden Zellen (Hepatocyten) ausgehen, auftritt.

Der Nachweis von Pyruvatkinase-Isoenzym Tumor-Typ M2 ist daher ein Test für einen Tumormarker, der ein sehr breites Spektrum von Tumoren erfaßt. Cooper, J.A., Reiss, N.A., Schwarz, R.J. and Hunter, T. Nature 302, 218-223 (1983); Reinacher, M. and Eigenbrodt, E. Virchows Archiv, 37, 79 79-88 (1981).

Reinacher und Eigenbrodt (Virchows Arch (cell Pathol.), 37 (1981), 79-88) offenbaren, daß Pyruvatkinase des Typs M2 in Tumorgewebe vorhanden ist und daß in malignen Tumoren hohe Mengen von Pyruvatkinase-Isoenzym Typ M2 gefunden werden. Es werden keine Antikörper gegen dieses Isoenzym beschrieben.

Weernink et al.(Chemical Abstracts, 109: 228157m) beschreiben die Erzeugung eines spezifischen Antikörpers gegen Pyruvatkinase Typ M2 unter Verwendung eines synthetischen Peptids. Dieser Antikörper kann nur zwischen den Isoenzymen M1 und M2, aber nicht zwischen dem Isoenzym Typ M2 und dem Isoenzym Typ Tumor M2 unterscheiden.

Van Erp et al. (Eur. J. Cell Biol. 47 (1988), 388-394) beschreiben die Erzeugung und Charakterisierung von monoklonalen Antikörpern gegen humane Pyruvatkinase Typ K. Diese Antikörper werden unter Verwendung von Pyruvatkinase Typ M2 aus normalem menschlichem Nierengewebe, nicht aus Tumorgewebe erzeugt. Diese Antikörper sind nicht in der Lage, zwischen Pyruvatkinase M2 aus Normalgewebe und Tumor M2 Pyruvatkinase zu unterscheiden.

Es wäre nun wüschenswert, Pyruvatkinase-Isoenzym Tumor-Typ M2 als Tumormarker im Blut, Plasma, Körperflüssigkeiten, Abstrichen oder Gewebe und/oder Gewebekultur (Biopsiematerial) spezifisch nachweisen zu können.

Der Nachweis dieses Tumormarkers kann immunhistologisch zum Nachweis von Mikrometastasen oder sogar einzelner Tumorzellen eingesetzt werden und kann im Sandwich-ELISA zur Untersuchung von Blut, Plasma, Körperflüssigkeiten, Abstrichen, Geweben und Gewebekultur verwandt werden. Zusätzlich gibt der Gehalt an Tumor-M2-PK einen Einblick in den jeweiligen Stoffwechselzustand der Tumorzelle bei Sauerstoffmangel (hypoxische Tumorzelle). Weiterhin lassen sich Schlüsse über die Purinsynthesefähigkeit und die NADPH- und GSA- Regenaration ziehen, die alle Ansatzpunkte von Chemotherapeutica sind oder den Stoffwechsel dieser Substanzen beeinflussen können.

Es war nun die Aufgabe der vorliegenden Erfindung, ein Verfahren zur quantitativen, spezifischen Bestimmung von Tumor M2-PK zur Verfügung zu stellen, das insbesondere den Nachweis sehr geringer Mengen an Tumor M2-PK ermöglicht, das sehr genau durchzuführen ist und das insbesondere den spezifischen Nachweis der Tumor-M2-PK ermöglicht, ohne daß ebenfalls vorhandene andere Pyruvatkinase-Isoenzyme und andere im Blut, Plasma, Körperflüssigkeiten oder Gewebe des menschlichen Organismus vorhandenen Proteine stören.

Diese Aufgabe wird gelöst durch einen Tumor-M2-PK-spezifischen Antikörper, der spezifisch bindefähig ist mit der Tumor-M2-PK und anderen Substanzen diskriminiert.

Antiseren, die die erfindungsgemäß verwendeten Antikörper enthalten, werden erhalten, indem man in den Organismus von Versuchstieren als Immunogen hochgereinigte Tumor-M2-PK oder Fragmente derselben einbringt.

Versuchstiere wie Mäuse, Ratten, Kaninchen, Ziegen oder Pferde werden in bekannter Weise immunisiert und so Antiseren mit polyklonalen Antikörpern erhalten. In einer bevorzugten Ausgestaltung werden in entsprechender Weise monoklonale Antikörper nach der Methode von G. Köhler und C. Milstein, Nature 256, 495-497 (1975) erzeugt.

Daher ist ein weiterer Gegenstand der Erfindung ein Verfahren zur Herstellung eines monoklonalen Antikörpers, der spezifisch mit Tumor-M2-PK bindefähig ist. Dieses Verfahren ist dadurch gekennzeichnet, daß man Mäuse oder Ratten mit hochgereinigter Tumor-M2-PK immunisiert, B-Lymphozyten aus der Milz der immunisierten Tiere mit Myelomzellen fusioniert, die gebildeten Hybridomazellen kloniert, die Hybridomazellen, die Tumor-M2-PK bindungsfähige Antikörper sezernieren, isoliert, kloniert und züchtet und den durch sie gebildeten monoklonalen Antikörper gewinnt.
Besonders bevorzugt wird eine Zellinie verwendet, die selbst kein Immunglobulin produziert.

Die erfindungsgemäß erhältlichen monoklonalen Antikörper reagieren nicht mit anderen Pyruvatkinase-Isoenzymen oder mit anderen Substanzen, sondern sind für Tumor-M2-PK spezifisch.

Ein weiterer Gegenstand der Erfindung ist die Verwendung des erfindungsgemäßen Tumor-M2-PK-spezifischen Antikörpers zur qualitativen und/oder quantitativen Bestimmung von Tumor-M2-PK. Es ist somit möglich, unter Verwendung dieses Antikörpers Tumor-M2-PK spezifisch in Körperflüssigkeiten oder Geweben, Abstrichen nachzuweisen.

Bei Versuchen zum Nachweis von Pyruvatkinase-Isoenzym im Blut, Plasma oder Serum wurden indirekte, kompetitive- und Sandwich-ELISA eingesetzt. Um von anderen nicht kalkulierbaren Serumfaktoren unabhängigzu sein, zeigte sich ein Sandwich-ELISA für die schnelle Diagnostik bei großem Probenumfang am geeignetesten.

Hierfür sind mindestens 2 verschiedene monoklonale Antikörper gegen unterschiedliche Epitope des Isoenzyms gerichtet, nötig. Mit solchen Tests lassen sich Isoenzymverschiebungen des Pyruvatkinase- Isoenzyms M2 z.B. im Serum insbesondere das Auftreten dieser Verschiebungen bei malignen Erkrankungen, nachweisen.

Bestimmungsverfahren nach dem Prinzip des Immunoassays sind weit verbreitet. Vorteil dieser Bestimmungsmethode ist ihre Genauigkeit und die Möglichkeit, sehr geringe Substanzmengen nachweisen zu können. Zur Durchführung der Bestimmung sind verschiedene Verfahrensvarianten möglich sowohl mit homogenen als auch mit heterogenen Phasen. Bei der Ausführungsform mit heterogener Phase wird einer der Rezeptoren an einen Träger gebunden. Beim Sandwich-Verfahren wird beispielsweise ein Rezeptor an den Träger gebunden, die Testlösung zugegeben, wobei das in der Testlösung enthaltene, zu bestimmende Antigen an den Rezeptor gebunden wird. Dann wird ein markierter Rezeptor zugegeben, der spetifisch mit dem Antigen oder dem Antigen-Antikörperkomlex reagiert. Über den markierten Antikörper kann dann die Menge an Antigen berechnet werden.

Für dieses allgemeine Prinzip gibt es viele Variationsmöglichkeiten. So kann beispielsweise eine Bestimmung mit drei Rezeptoren erfolgen, wobei einer der drei Rezeptoren in heterogener Phase vorliegt und die anderen beiden Rezeptoren löslich sind wobei einer der beiden löslichen Rezeptoren markiert ist, während der andere unmarkiert ist. Der lösliche Rezeptor ist dann gegen den nicht markierten löslichen Rezeptor gerichtet.

Die Verwendung des erfindungsgemäßen Tumor-M2-PK- spezifischen Antikörpers zur selektiven quantitativen Bestimmung von Tumor-M2-PK nach dem Prinzip des Immunoassays durch Inkubation mit mindestens zwei verschiedenen Rezeptoren, von denen der erste Rezeptor R1 in fester Phase vorliegt und mit der Tumor-M2-PK und einem Rezeptor bindefähig ist und mindestens ein weiterer Rezeptor R2, der mit der Tumor-M2-PK und einem Rezeptor bindefähig ist, in flüssiger Phase gelöst und wobei ein Rezeptor R2 eine Markierung trägt, Trennung der festen von der flüssigen Phase und Messung der Markierung in einer der Phasen, ist dadurch gekennzeichnet, daß man als einen der Rezeptoren einen Tumor-M2-PK- spezifischen Antikörper verwendet und der zwischen Tumor-M2-PK und zwischen anderen Pyruvatkinase-Isoenzymen diskriminiert.

Zur Durchführung dieses Verfahrens werden mindestens zwei verschiedene Rezeptoren verwendet. Einer von diesen Rezeptoren ist spezifisch mit Tumor-M2-PK bindefähig. Mindestens ein weiterer Antikörper muß ebenfalls mit Tumor-M2-PK bindefähig sein. Hier kann ein Antikörper verwendet werden, der Tumor-M2-PK unspezifisch bindet und auch mit anderen Pyruvatkinase-Isoenzymen eingeht. Bevorzugt wird jedoch auch hier ein Rezeptor verwendet, der spezifisch bindefähig mit Tumor-M2-PK ist.

Einer der beiden Rezeptoren wird an eine Festphase gebunden. Die Bindung an die Festphase erfolgt in an sich bekannter Weise und ist dem Fachmann bekannt. Weiterhin wird mindestens ein weiterer Rezeptor verwendet, der in löslicher Form vorliegt.

Dieser weitere Rezeptor trägt eine Markierung. Werden mehrere weitere Rezeptoren R2 verwendet, so trägt nur einer von diesen eine Markierung. Die Markierung des Rezeptors erfolgt in an sich üblicher Weise und ist dem Fachmann bekannt.

Die Markierung erfolgt in an sich bekannter Weise bevorzugt durch eine radioaktive Verbindung, ein Enzym, eine chemilumineszente oder fluoreszierende Verbindung. Besonders bevorzugt wird die Markierung mit einem Enzym vorgenommen, insbesondere mit Peroxidase oder Phosphatase. Die Markierung mit einem Enzym erlaubt in einer besonderen Testgestaltungsvariante, diesen Antikörper, gemäß dem Stand der Technik in einem zweiten Enzymamplitikationssystem einzusetzen. Stanley, C.J., Paris, F., Plumb, A., Webb, A. Johannsson, A. American Biotechnology Laboratory: May/June 1985; Self, C.H. J. Immunol. Meth. (1985), in press; Johannsson, A., Stanley, C.J., Self, C.H. Clin. Chem. Acta (1985), in press.

In einer besonders bevorzugten Ausführungsform des Verfahrens wird nun entweder ein unspezifisch mit Tumor-M2-PK bindetähiger Rezeptor oder bevorzugt ein spezifisch mit Tumor-M2-PK bindefähiger Rezeptor an eine Festphase gebunden. Dieser an die Festphase gebundene Rezeptor wird anschließend mit der die zu bestimmende Tumor-M2-PK enthaltenden Lösung und einem Antikörper, der spezifisch mit der Tumor-M2-PK bindefähig ist, in löslicher Form vorliegt und eine Markierung trägt, inkubiert. Ist der an die Festphase gebundene Rezeptor ein unspezifisch mit Tumor-M2-PK bindefähiger, so lagern sich an der Festphase nicht nur Tumor-M2-Pk sondern auch andere Pyruvatkinase-Isoenzyme an. Der zweite Antikörper, der spezifisch mit Tumor-M2-PK bindefähig ist, lagert sich dann nur an Tumor-M2-PK, so daß nur die Tumor-M2-PK spezifisch einen markierten Antikörper tragen, während die anderen Pyruvat-Isoenzyme nicht markiert werden. Auf diese Weise ist es nach Trennung der festen von der flüssigen Phase möglich, über die Messung der Markierung den Gehalt an Tumor-M2-PK zu bestimmen.

Wird als an die Festphase gebundener Rezeptor ein mit Tumor-M2-PK spezifisch bindefähiger Rezeptor verwendet, so wird an der Festphase spezifisch nur Tumor-M2-PK gebunden und bei der Inkubation mit dem löslichen Antikörper reagiert auch dieses wiederum spezifisch mit der Tumor-M2-PK. Da somit praktisch keine Bindung mit anderen Pyruvatkinase-Isoenzyme an die Festphase erfolgt, ist dieses Verfahren noch spezifischer und erlaubt daher sehr genaue Bestimmungen.

In einer weiteren besonders bevorzugten Ausführungsform des erfindungsgemäßen Verfahrens wird ein gegen die Tumor-M2-PK spezifisch bindefähiger Antikörper an einem Träger immobilisiert. Mit diesem immobilisierten Antikörper wird dann die zu bestimmende Tumor-M2-PK enthaltende Lösung und ein mit Tumor-M2-PK bindefähiger Rezeptor, vorzugsweise ein spezifisch mit Tumor-M2-PK bindefähiger Rezeptor inkubiert.

Dabei wird an der Festphase praktisch ausschließlich Tumor-M2-PK gebunden, andere Pyruvatkinase-Isoenzyme bleiben in Lösung. Weiterhin lagert sich die Tumor-M2-PK der lösliche, markierte mit Tumor-M2-PK bindefähiger Antikörper an. Nach Trennung der festen von der flüssigen Phase kann wiederum über die Markierung der Gehalt an Tumor-M2PK sehr genau bestimmt werden.

Weitere, dem Fachmann bekannte Verfahrensvarianten mit drei Rezeptoren sind ebenfalls unter Verwendung des spezifisch mit der Tumor-M2-PK spezifisch bindefähigen Antikörpers möglich und bedürfen hier keiner weiteren Ausführungen.

Von den für die Durchführung des erfindungsgemäßen Verfahrens verwendeten Antikörpern ist bevorzugt mindestens einer ein monoklonaler Antikörper. Besonders bevorzugt werden als Rezeptoren nur monoklonale Antikörper verwendet.

Ein weiterer Gegenstand der Erfindung ist ein Reagenz zur selektiven Bestimmung von Tumor-M2-PK, enthaltend einen an eine Festphase gebundenen Rezeptor R1, der mit Tumor-M2-PK und einem Rezeptor bindefähig ist und mindestens einen in löslicher Phase vorliegenden Rezeptor R2, der mit Tumor-M2-PK und einem Rezeptor bindefähig ist, wobei ein löslicher Rezeptor R2 eine Markierung trägt, das dadurch gekennzeichnet ist, daß einer der Rezeptoren ein Antikörper ist, der spezifisch mit Tumor-M2-PK bindefähig ist.

Der Tumor-M2-PK spezifisch bindefähige Antikörper kann entweder an die Festphase gebunden vorliegen oder aber als löslicher, markierter oder unmarkierter Rezeptor verwendet werden. Bevorzugt ist dieser Antikörper ein monoklonaler Antikörper. Besonders bevorzugt sind alle verwendeten Rezeptoren monoklonale Antikörper.

Weiterhin kann der erfindungsgemäße Tumor-M2-PKspezifische Antikörper vorteilhaft zur qualitativen Bestimmung von Tumor-M2-PK- Ansammlungen im menschlichen Blutkreislauf verwendet werden.

Dazu wird bevorzugt der erfindungsgemäße Tumor-M2-PKspezifische Antikörper, besonders bevorzugt der erfindungsgemäß hergestellte monoklonale Tumor-M2-PKspezifische Antikörper markiert, in den Blutkreislauf injiziert und durch geeignete Mittel sichtbar gemacht.

Besonders bevorzugt wird Patienten der erfindungsgemäße Tumor-M2-PKspezifische Antikörper, der radioaktiv markiert wurde, injiziert. Der Antikörper reichert sich an den Ansammlungen z.B. Metastasen oder Mikrotumoren an und kann mit einer gamma-Kamera, oder ähnlichen, dem Fachmann bekannten, Nachweisegeräten nachgewiesen werden. Auf diese Weise kann nicht nur die Lage der Tumoren oder Metastasen, sondern auch ihre Größe ermittelt werden.

Weiterhin kann der Tumor-M2-PKspezifische Antikörper dazu benutzt werden, Medikamente, die maligne Zellen z.B. Metastasen,oder Mikrotumoren lysieren können, gezielt zu diesen Mikrotumoren zu führen, indem man die entsprechenden Medikamente an die Antikörper koppelt. Als lysierende Substanzen können beispielsweise verschiedene Cytostatika, Toxine verwendet werden.

Eine Anwendungsvariante ist die Verwendung menschlicher monoklonaler Antikörper.

Da der erfindungsgemäß verwendete Antikörper nicht mit anderen Pyruvatkinase-Isoenzymen reagiert, führt diese Therapie nicht zu einer nennenswerten Abtötung nicht maligner Zellen, welches immer die Folge einer herkömmlichen Chemotherapie ist.

Weiterhin erlauben die erfindungsgemäß hergestellten Antikörper die Verlaufskontrolle einer Chemo- und/oder Radio-Therapie.

Erfindungsgemäß wird ein Antikörper zur Verfügung gestellt, der spezifisch mit Tumor-M2-PK reagiert und andere Pyruvatkinase-Isoenzyme nicht bindet. Mit diesem Antikörper ist es möglich, Tumor-M2-PK im Blut oder anderen Körperflüssigkeiten, Abstrichen spezifisch sowohl qualitativ als auch quantitativ nachzuweisen. Der erfindungsgemäße Antikörper erlaubt es, genaue Aussagen über den Tumor-M2-PK in diesen Proben zu machen und andererseits Mikrotumore (Metastasen), die sich im Körper gebildet haben und zu großen Tumoren führen können, zu ermitteln. Auch ist es möglich, gebildete Tumore spezifisch abzutöten.

Weiterhin ist es möglich den Grad der Malignität (Bösartigkeit), Digität eines Tumors oder Tumorgewebes an Hand der gemessenen Menge an Tumor-M2-PK zu bestimmen, da der Gehalt an Tumor-M2-PK in den Tumorzellen mit der Malignität dieser Zellen korrelliert.

Die Erfindung wird noch durch die folgenden Beispiele erläutert.

### Herstellung monoklonaler Antikörper

### Beispiel 1

Hochgereinigte menschliche Tumor-M2-PK wird in PBS gelöst und zu gleichen Teilen mit Freund-Adjuvanz gemischt. Jeweils 100ug dieses Gemisches werden in 6 bis 8 Wochen alte Balb/c Mäuse i.p. und s.c. injiziert. Diese Injektionen werden zweimal im Abstand von 3 bis 4 Wochen wiederholt. Nach obengenanntem Schema mit hochgereinigter Tumor-M2-PK immunisierte Mäuse erhalten 3 Tage vor Entnahme der Milz eine i.v. Injektion von 100ug hochgereinigter Tumor-M2-PK, welches in PBS gelöst wird.

Etwa 100 Mill. Zellen von der Milz einer immunisierten Maus werden mit 50 Mill. Myelomazellen (x63-Sp8-653, eine Zelllinie, die kein Immunglobulin synthetisiert; zu beziehen bei The Salk Institute, Cell Distribution Center, San Diego CA 92112, USA) in Gegenwart von Polyethylenglykol (MG 3400) fusioniert. Fusionierte Zellen werden auf 4 Platten, die jeweils 24 Vertiefungen enthalten, ausgesät. Jede dieser Vertiefungen enthält 50 Mill. Milzzellen unimmunisierter syngener Mäuse in Nährmedium, welches Hypoxantin, Aminopterin und Thymidin enthält.

Die Antikörper enthaltenden Überstände dieser fusionierten Zellen (Hybridoma) werden 10 bis 14 Tage später mittels ELISA, Westernblott, Gefrierschnitt, Paraffinschnitt bezüglich ihrer Spezifität gegen folgendes Antigen getestet: hochgereinigte, menschliche Tumor-M2-PK
Um monoklonale Antikörper zu erhalten, die nur gegen Tumor-M2-PK gerichtet sind, werden Hybridomazellen, deren Überstände keine gegen andere Pyruvatkinase-Isoenzyme gerichteten Antikörper enthalten, zweimal kloniert.

### Bestimmung von Tumor-M2-PK in Plasma mit monoklonalen Tumor-M2-PK- spezifischen Antikörpern.

### Beispiel 2

Es wird Tumor-M2-PK im Plasma mit einem ELISA bestimmt. Hierzu werden gemäß Beispiel 1 erhaltene monoklonale, Tumor-M2-PK-spezifische Antikörper aus M₂PK, die in PBS, pH 7,2, gelöst, an Polystyrol als Träger immobilisiert. Nach einem Waschschritt wird verdünntes Plasma, welches Tumor-M2-PK enthält, dazugegeben. Das Plasma wird in einem Puffer der PBS 5 mmol EDTA, 2umol EACA 200 E Aprotinin/1 und 0,2% Tween 20 enthält, verdünnt.

Nach einem Waschschritt im PBS, 0,1% Tween 20 wird die an den Antikörper gebundeneTumor-M2-PK mit einem polyklonalen Antikörper, der sowohl Tumor-M2-PK als auch andere Pyruvatkinase-Isoenzyme bindet und an den Phosphatase gekoppelt ist, der gelöst ist in PBS, das 0,2% Tween 20 enthält und einen pH von 7,2 hat, eine Stunde bei Raumtemperatur inkubiert. Nach einem erneuten Waschschritt wird durch Zugabe von p-Nitrophenylphosphatdinatriumhexahydrat eine Änderung der optischen Dichte in den Reaktionsgefäßen gemessen, in denen die monoklonalen Antikörper mit der Tumor-M2-PK reagiert haben.

### Bestimmung von Tumor-M2-PK in Plasma mit zwei verschiedenen Tumor-M2-PK- spezifischen Antikörpern.

### Beispiel 3

Der monoklonale Antikörper aus M₂PK, der gegen Tumor-M2-PK gerichtet ist, wird, wie im Beispiel 2 beschrieben, an einen Träger fixiert. Nach einem Waschschritt wird Plasma, das Tumor-M2-PK enthält, mit dem monoklonalen Antikörper unter denselben Bedingungen wie im Beispiel 2 inkubiert. Nach einem weiteren Waschschritt wird die Tumor-M2-PKspezifische durch einen zweiten erfindungsgemäßen Tumor-M2-PKspezifischen monoklonalen Antikörper aus M₂PK II, der auch gegen die Tumor-M2-PK gerichtet ist, detektiert. Dieser zweite Tumor-M2-PK- spezifische Antikörper trägt Peroxydase kovalent gebunden. Nach einem Waschschritt wird nach Zugabe von ABTS als Substrat für Peroxydase eine Änderung der optischen Dichte gemessen.

### Beispiel 4

Es wurde wie in Beispiel 3 beschrieben verfahren, jedoch wurde an den zweiten Tumor-M2-PKspezifischen Antikörper aus M₂PK II statt eines Enzyms Biotin gekoppelt. Vor Substratzugabe wurde dann Peroxidase-konjugiertes Avidin oder Peroxydase-konjigiertes Streptavidin zugesetzt. Die so gewählte Bestimmung von Tumor-M2-PK erlaubt ganz spezifisch nur Tumor-M2-PK zu bestimmen. Andere Pyruvatkinase-Isoenzyme, die in der Lösung enthalten sind, stören die erfindungsgemäße Bestimmung der Tumor-M2-PK nicht.

### Beispiel 5

Ein gemäß Beispiel 1 erhaltener, Tumor-M2-PKspezifischer Antikörper aus M₂PK wird radioaktiv markiert. Dieser markierte Antikörper wurde zu einer Lösung von 1% BSA in PBS zugegeben, die Zellen eines Tumors enthielt. Der Anteil der an die Tumorzellen gebundenen Antikörper wurde in einem Szintillationszähler bestimmt. Zum Vergleich wurden zu einer Tumorzellen enthaltenden Pufferlösung radioaktiv markierte Antikörper zugegeben, die nicht mit Tumor-M2-PK reagieren. Ein Vergleich der Ergebnisse der Szintillometrie zeigte, daß die erfindungsgemäßen Antikörper spezifisch an Tumorzellen binden, während die Kontrollantikörper keine Bindung mit den Tumorzellen eingingen.

## Patentansprüche

1. Pyruvatkinase(ATP: pyruvate 2-O-phosphotransferase. EC 2.7. 1.40)-Isoenzym Typ Tumor M2 (Tumor M2-PK)-spezifischer Antikörper, diskriminierend zwischen anderen Pyruvatkinase-Isoenzymen insbesondere M1, L und R-Typ.

2. Verfahren zur Gewinnung eines monoklonalen Antikörpers, der spezifisch an Pyruvatkinase-Isoenzym Typ Tumor M2 (Tumor M2-PK) bindet und zwischen anderen Pyruvatkinase-Isoenzymen(M1,L und R-Typ diskriminiert, dadurch gekennzeichnet, daß man Mäuse oder Ratten mit dem hochgereinigten Pyruvatkinase-Isoenzym Tumor M2 (Tumor M2-PK) immunisiert, B-Lymphozyten aus der Milz der immunisierten Tiere mit Myelomzellen fusioniert, die gebildeten Hybridomazellen kloniert, die Klone, die Pyruvatkinase-Isoenzym Tumor M2 (Tumor M2-PK)- bindende Antikörper produzieren, isoliert, auf Diskriminierung zwischen anderen Pyruvatkinase-Isoenzymen selektioniert und züchtet und den durch sie gebildeten monoklonalen Antikörper gewinnt.

3. Verfahren nach Anspruch 2, dadurch gekennzeichnet, daß man Myelomzellen verwendet, die selbst kein Immunglobulin produzieren.

4. Verfahren nach Anspruch 2 oder 3 dadurch gekennzeichnet, daß man als Immunogen das hochgereinigte, menschliche Pyruvatkinase-Isoenzym Typ Tumor M2 (Tumor M2-PK) verwendet.

5. Verfahren nach Anspruch 2 bis 4, dadurch gekennzeichnet, daß man als Immunogen ein synthetisches Teilpeptid mit der Sequenz: -Arg-Arg-Leu-Ala-Pro-Ile-Thr-Ser-Asp-Pro-Thr-Glu-Ala-Ala-Ala-des menschlichen Pyruvatkinase-Isoenzyms Typ Tumor M2 (Tumor M2-PK) verwendet, das wahlweise über einen Spacer an ein Trägerpeptid gebunden ist.

6. Verfahren nach Anspruch 5 dadurch gekennzeichnet, daß als Trägerpeptid ein Albumin oder ein Hämocyanin verwendet wird.

7. Verwendung des Tumor M2-PK spezifischen Antikörpers nach Anspruch 1 zur selektiven Bestimmung der Tumor M2-PK nach dem Prinzip des Immunoassays durch Inkubation mit mindestens zwei verschiedenen Rezeptoren, von denen der erste Rezeptor R1 in fester Phase vorliegt und mit Tumor M2-PK bindefähig ist und mindestens ein weiterer Rezeptor R2, der mit Tumor M2-PK und einem Rezeptor bindefähig ist, in flüssiger Phase gelöst vorliegt und wobei ein Rezeptor R2 eine Markierung trägt, Trennung der festen von der flüssigen Phase und Messung der Markierung in einer der Phasen, dadurch gekennzeichnet, daß man als einen der Rezeptoren einen Antikörper verwendet, der spezifisch mit Tumor M2-PK bindefähig ist und zwischen anderen Pyruvatkinase-Isoenzymen diskriminiert.

8. Verwendung nach Anspruch 7, dadurch gekennzeichnet, daß man als an die feste Phase gebundenen Antikörper einen Antikörper verwendet, der spezifisch mit der Tumor M2-PK bindefähig ist und als löslichen Rezeptor einen anderen mit Tumor M2-PK bindefähigen Antikörper verwendet, der eine Markierung trägt.

9. Verwendung nach einem der Ansprüche 7 bis 8, dadurch gekennzeichnet, daß die Markierung eine radioaktive Verbindung, ein Enzym, eine chemoluminiszierende oder fluoreszierende Verbindung ist.

10. Verwendung nach einem der Ansprüche 7 bis 9, dadurch gekennzeichnet, daß der Antikörper, der spezifisch mit der Tumor M2-PK bindefähig ist, ein monoklonaler Antikörper ist.

11. Verwendung des Antikörpers nach Anspruch 1 zur qualitativen und quantitativen in vitro Bestimmung der Tumor-M2-PK in Blut, Plasma, Serum und Gewebe, Gewebekultur bzw. Gewebeschnitten und Biopsiematerial (Immunhistologie: Paraffin- und/oder Gefrierschnitten).

12. Reagenz zur selektiven, quantitativen Bestimmung von Tumor-M2-PK, enthaltend einen an eine Festphase gebundenen Rezeptor R1, der mit Tumor M2-PK und einem Rezeptor bindefähig ist und mindestens einen in löslicher Phase vorliegenden Rezeptor R2, der mit dem Tumor-M2-PK und einem Rezeptor bindefähig ist und wobei ein löslicher Rezeptor R2 eine Markierung trägt, dadurch gekennzeichnet, daß einer der Rezeptoren ein Antikörper ist, der spezifisch mit der Tumor-M2-PK bindefähig ist und zwischen anderen (verschiedenen) Pyruvatkinase-Isoenzymen diskriminiert.

## Claims

1. Antibody specific for the pyruvate kinase (ATP: pyruvate 2-O-phosphotransferase EC 2.7.1.40) tumour M2-type isoenzyme (tumour M2-PK) which discriminates between other pyruvate kinase isoenzymes and in particular the M1, L and R type.

2. Process for obtaining a monoclonal antibody which binds specifically to the pyruvate kinase tumour M2-type isoenzyme (tumour M2-PK) and discriminates between other pyruvate kinase isoenzymes (M1, L and R type), wherein mice or rats are immunized with the highly purified pyruvate kinase tumour M2 isoenzyme (tumour M2-PK), B lymphocytes from the spleen of the immunized animals are fused with myeloma cells, the hybridoma cells which are formed are cloned, the clones that produce antibodies binding the pyruvate kinase tumour M2 isoenzyme (tumour M2-PK) are isolated, selected for discrimination between other pyruvate kinase isoenzymes and cultured and the monoclonal antibodies which are formed by them are isolated.

3. Process as claimed in claim 2, wherein myeloma cells are used which do not themselves produce immunoglobulin.

4. Process as claimed in claim 2 or 3, wherein a highly purified human tumour-M2-type pyruvate kinase isoenzyme (tumour M2-PK) is used as the immunogen.

5. Process as claimed in claim 2 to 4, wherein a synthetic partial peptide with the sequence: -Arg-Arg-Leu-Ala-Pro-Ile-Thr-Ser-Asp-Pro-Thr-Glu-Ala-Ala-Ala of the human tumour-M2-type pyruvate kinase isoenzyme (tumour M2-PK) is used as the immunogen which is optionally bound to a carrier peptide via a spacer.

6. Process as claimed in claim 5, wherein an albumin or a haemocyanin is used as the carrier peptide.

7. Use of the tumour-M2-PK-specific antibody as claimed in claim 1 for the selective determination of tumour M2-PK according to the immunoassay principle by incubation with at least two different receptors of which the first receptor R1 is present in a solid phase and is capable of binding to tumour M2-PK and at least one additional receptor R2 which is capable of binding to tumour M2-PK and to a receptor and is present dissolved in a liquid phase in which receptor R2 carries a label, separation of the solid from the liquid phase and measurement of the label in one of the phases, wherein an antibody which is capable of specific binding to tumour M2-PK and discriminates between other pyruvate kinase isoenzymes is used as one of the receptors.

8. Use as claimed in claim 7, wherein an antibody is used as the antibody bound to the solid phase which is capable of specific binding to tumour M2-PK and another antibody capable of binding to tumour M2-PK is used as the soluble receptor which carries a label.

9. Use as claimed in one of the claims 7 to 8, wherein the label is a radioactive compound, an enzyme, a chemiluminescent or fluorescent compound.

10. Use as claimed in one of the claims 7 to 9, wherein the antibody which is capable of specific binding to tumour M2-PK is a monoclonal antibody.

11. Use of the antibody as claimed in claim 1 for the qualitative and quantitative in vitro determination of tumour M2-PK in blood, plasma and tissue, tissue culture or tissue sections and biopsy material (immunohistology: paraffin and/or frozen sections).

12. Reagent for the selective quantitative determination of tumour M2-PK containing a receptor R1 bound to a solid phase which is capable of binding to tumour M2-PK and to a receptor and at least one receptor R2 present in the soluble phase which is capable of binding to tumour M2-PK and to a receptor and in which a soluble receptor R2 carries a label, wherein one of the receptors is an antibody which is capable of specific binding to tumour M2-PK and discriminates between other (different) pyruvate kinase isoenzymes.

## Revendications

1. Anticorps ayant une spécificité pour l'isoenzyme de la pyruvate-kinase(ATP: pyruvate-2-O-phosphotransférase, EC 2.7.1.40) de type M2 de tumeur (M2-PK tumorale) en la distinguant parmi d'autres isoenzymes de la pyruvate-kinase, en particulier de type M1, L et R.

2. Procédé d'obtention d'un anticorps monoclonal qui se lie de façon spécifique à l'isoenzyme de la pyruvate-kinase de type M2 de tumeur (M2-PK tumorale) et qui la distingue parmi d'autres isoenzymes de la pyruvate-kinase (de type M1, L et R), caractérisé en ce que l'on immunise des souris ou des rats avec l'isoenzyme de la pyruvate-kinase M2 tumorale (M2-PK tumorale), on fusionne des lymphocytes B issus de la rate des animaux immunisés avec des cellules de myélome, on clone les cellules d'hybridomes formées, on isole les clones qui produisent des anticorps se liant avec l'isoenzyme de pyruvate-kinase M2 tumorale (M2-PK tumorale), on les sélectionne par rapport à leur capacité de la distinguer parmi d'autres isoenzymes de la pyruvate-kinase, on les cultive et on récupère les anticorps monoclonaux qu'ils produisent.

3. Procédé selon la revendication 2, caractérisé en ce que l'on utilise des cellules de myélome qui ne produisent pas elles-mêmes d'immunoglobuline.

4. Procédé selon la revendication 2 ou 3, caractérisé en ce que l'on utilise comme immunogène l'isoenzyme de la pyruvate-kinase de type M2 de tumeur (M2-PK tumorale) humaine hautement purifiée.

5. Procédé selon les revendications 2 à 4, caractérisé en ce que l'on utilise comme immunogène une fraction peptidique synthétique ayant la séquence -Arg-Arg-Leu-Ala-Pro-Ile-Thr-Ser-Asp-Pro-Thr-Glu-Ala-Ala-Ala- de l'isoenzyme de la pyruvate-kinase de type M2 de tumeur (M2-PK tumorale) humaine et qui, selon les besoins, est liée à un peptide support par l'intermédiaire d'un espaceur.

6. Procédé selon la revendication 5, caractérisé en ce que l'on utilise comme peptide support une albumine ou une hémocyanine.

7. Utilisation de l'anticorps spécifique anti-M2-PK tumorale selon la revendication 1 pour le dosage spécifique de la M2-PK tumorale selon le principe de l'essai immunologique par incubation avec au moins deux récepteurs différents, dont le premier récepteur R1 se trouve en phase solide et est capable de se lier à la M2-PK tumorale, et au moins un autre récepteur R2, qui se lie à la M2-PK tumorale et à un récepteur, est dissous en phase liquide, au moins un récepteur R2 portant un marquage, séparation de la phase solide de la phase liquide et mesure de la quantité marquée dans l'une des phases, caractérisée en ce que l'on utilise comme l'un des récepteurs un anticorps qui se lie de façon spécifique à la M2-PK tumorale et qui la distingue parmi d'autres isoenzymes de la pyruvate-kinase.

8. Utilisation selon la revendication 7, caractérisée en ce que l'on utilise comme anticorps lié à la phase solide un anticorps qui se lie de façon spécifique à la M2-PK tumorale, et comme récepteur soluble un autre anticorps se liant à la M2-PK tumorale et qui porte un marquage.

9. Utilisation selon l'une des revendications 7 à 8, caractérisée en ce que le marquage est constitué par un composé radioactif, une enzyme, ou un composé chimioluminescent ou fluorescent.

10. Utilisation selon l'une des revendications 7 à 9, caractérisée en ce que l'anticorps qui se lie de façon spécifique à la M2-PK tumorale est un anticorps monoclonal.

11. Utilisation de l'anticorps selon la revendication 1 pour la détermination quantitative et qualitative in vitro de la M2-PK tumorale dans le sang, le plasma, le sérum et les tissus, une culture de tissu ou des coupes de tissus et des produits de biopsie (immunohistologie: coupes paraffiniques et/ou coupes congelées).

12. Réactif pour la détermination sélective, quantitative de la M2-PK tumorale, contenant un récepteur R1 lié à la phase solide, lequel récepteur R1 est capable de se lier à la M2-PK tumorale et à un récepteur et contenant au moins un récepteur R2 présent dans la phase soluble, lequel récepteur R2 peut se lier à la M2-PK tumorale et à un récepteur et ce en quoi un récepteur soluble R2 porte un marquage, caractérisé en ce que l'un des récepteurs est un anticorps qui est capable de se lier de façon spécifique à la M2-PK tumorale et qui la distingue parmi d'autres isoenzymes de la pyruvate-kinase (différentes).
